# EUROPEAN PATENT SPECIFICATION

(11) **EP 4 474 807 B1**
(45) Date of publication and mention of the grant of the patent: **30.09.2026**
(21) Application number: 24179827.1
(22) Date of filing: 04.06.2024
(51) Int. Cl.: G01N 27/00, A61B 5/05

(54) **ELECTROMAGNETIC TOMOGRAPHY METHOD FOR DETECTING ANOMALIES IN A BODY**
ELEKTROMAGNETISCHES TOMOGRAPHIE-VERFAHREN ZUR ERKENNUNG VON ANOMALIEN IN EINEM KÖRPER
MÉTHODE DE TOMOGRAPHIE ÉLECTROMAGNETIQUE POUR DÉTECTER DES ANOMALIES DANS UN CORPS

(30) Priority: 08.06.2023 IT 202300011745
(43) Date of publication of application: 11.12.2024
(73) Proprietor: Tamburrino, Antonello, 84084 Fisciano (SA) (IT); Mottola, Vincenzo, 03030 Villa Santa Lucia (FR) (IT)
(72) Inventor: Tamburrino, Antonello, 84084 Fisciano (SA) (IT); Mottola, Vincenzo, 03030 Villa Santa Lucia (FR) (IT)
(74) Representative: Studio Torta S.p.A.

(56) References cited:
- FLAVIO CALVANO ET AL: "Fast methods for shape reconstruction in Electrical Resistance Tomography", NDT&E INTERNATIONAL, ELSEVIER, AMSTERDAM, NL, vol. 46, 24 October 2011 (2011-10-24), pages 32 - 40, XP028122300, ISSN: 0963-8695, [retrieved on 20111112], DOI: 10.1016/J.NDTEINT.2011.10.007
- BORIJINDARGOON NARONG ET AL: "MUSIC-Like Algorithm for Source Localization in Electrical Impedance Tomography", IEEE TRANSACTIONS ON INDUSTRIAL ELECTRONICS, IEEE SERVICE CENTER, PISCATAWAY, NJ, USA, vol. 66, no. 6, 1 June 2019 (2019-06-01), pages 4661 - 4671, XP011707471, ISSN: 0278-0046, [retrieved on 20190131], DOI: 10.1109/TIE.2018.2863196
- ANTONELLO TAMBURRINO ET AL: "The Monotonicity Principle for Magnetic Induction Tomography", ARXIV.ORG, CORNELL UNIVERSITY LIBRARY, 201 OLIN LIBRARY CORNELL UNIVERSITY ITHACA, NY 14853, 21 July 2021 (2021-07-21), XP091002321

## Description

### TECHNICAL FIELD

The present invention relates to an electromagnetic tomography method for detecting position and shape of anomalies in a body of interest characterized by a certain electrical and/or magnetic property.

In particular, the present invention finds advantageous, but not exclusive application in electrical resistance tomography, to which the following description will make explicit reference without thereby losing in generality.

### CONTEXT

Electromagnetic tomography is a technique used according to various subclasses, such as for example electrical resistance or impedance tomography, electrical capacitance tomography and magnetic inductance tomography, in various application fields, such as for example the medical field for the research of breast and lung cancers, the oil industry for the search for deposits in the subsoil, the chemical process industry for the analysis of two-step mixtures, civil engineering for the search for fractures in structures, and in the field of nanomaterials to verify the variations in electrical conductivity when a wafer of such materials is subjected to chemical-physical processes.

The reconstruction of the images of bodies or materials to be investigated by electromagnetic tomography is normally carried out with iterative type algorithms that are performed by a computer.

In a nutshell, these algorithms iteratively modify a physical property of interest of the material of a virtual body, i.e. a numerical model of a real body, as long as values of numerically calculated electrical quantities on the virtual body are not sufficiently close to values of the same electrical quantities measured on the real body to be investigated, which will hereinafter be called the body of interest. Aim of the iterative algorithm is to identify in the volume of the body of interest an anomaly of said physical property of the material. In other words, given the same geometry for the body of interest and the virtual body and a certain electrical excitation applied on a frontier of the body of interest, said iterative algorithms are adapted to solve a minimum problem between measurements on the frontier of the body of interest and the values calculated on a frontier of the virtual body in which said calculated values are obtained, typically, by means of a numerical model, for example a finite element one.

In particular, each step of the aforesaid iterative algorithms provides for calculating the values of an electrical quantity on the frontier of the virtual body and this calculation consists of the numerical solution of a physical model that describes stationary electrical conduction and which is defined by Laplace equation. By assuming that the physical property of the material is linear and by applying the finite element method, the solution of the physical model for the virtual body is assimilated to the solution of a system of linear equations. The dimension of the system of linear equations is bound to the number of nodes N of the mesh with which the volume of the virtual body is discretized.

One possible method for the numerical solution of a linear system is Gauss's method which has a computational cost equal to $\frac{1}{3} {N}^{3} + M ⋅ O \left({N}^{2}\right) ,$ where 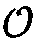 (*N*²) is an amount less than or equal to the product of a constant multiplied by N² and M is the number of known terms to be processed, which corresponds to the number of independent excitations given to the system. Given a number K of iterations of the iterative algorithm, the overall computational cost *Comp* can be estimated as follows: $Comp = \frac{K}{3} {N}^{3} + K ⋅ M ⋅ O \left({N}^{2}\right) .$

Considering that optimistically N can be equal to or greater than 1000 and that K is not a modest number, due to the misplaced nature of the inverse imaging problem, then the computational cost of the iterative algorithms described above in the field of electromagnetic tomography is very relevant and decidedly incompatible with a reconstruction of the images in real time.

A paper by Flavio Calvano et al., titled "Fast methods for shape reconstruction in Electrical Resistance Tomography", published by ELSEVIER on 12 November 2011, describes Electrical Resistance Tomography (ERT) methods and techniques aimed to reconstruct the spatial distribution of the conductivity of a material starting from the knowledge of boundary measurements such as, for instance, the Neumann-to-Dirichlet map. In particular, the paper presents a comparison between three fast non-iterative reconstruction methods for locating inclusions in an otherwise homogeneous material (for both 2D and 3D geometries). These methods, potentially, are candidate for real-time applications.

A paper by Narong Borijindargoon et al., titled "MUSIC-Like Algorithm for Source Localization in Electrical Impedance Tomography", published by IEEE Transactions on Industrial Electronics, vol. 66, n. 6, on June 2019, discloses an algorithm called MUSIC-like, which has high resolution performance comparable to MUSIC, for Electrical Impedance Tomography (EIT) application. The algorithm is formulated under the beamforming framework and, therefore, does not require an estimation of model order from the covariance matrix. Simulation results show that the proposed algorithm is capable of obtaining high resolution performance under various noise levels. The paper also discloses a 8-electrode EIT system prototype which was built using the proposed algorithm, and experimental results confirm the high resolution performance capability of the algorithm.

A paper by Antonello Tamburrino et al., titled "The Monotonicity Principle for Magnetic Induction Tomography", published by the Inverse Problems Journal, vol. 37, on 21 July 2021, discloses the reconstruction of the electrical conductivity from the free response of the system in the magneto-quasi-stationary (MQS) limit. The MQS limit corresponds to a diffusion PDE. A key role is played by the Monotonicity Principle (MP), that is a monotone relation connecting the unknown material property to the (measured) free-response. The MP is relevant as the basis of noniterative and real-time imaging methods. The paper discloses a monotonic relationship between the electrical resistivity and the time constants characterizing the free response for MQS systems. The key result is the representation of the induced current density through a modal representation. The main result is based on the analysis of an elliptic eigenvalue problem obtained from the separation of variables.

### SUMMARY

Aim of the present invention is to provide an electromagnetic tomography method, which is free from the drawbacks described above and, at the same time, is easy and economical to implement.

In accordance with the present invention there is provided an electromagnetic tomography method as defined in the appended claims.

### BRIEF DESCRIPTION OF THE DRAWINGS

The present invention will now be described with reference to the attached drawings, which show a non-limiting embodiment example thereof, wherein:
- Figure 1 shows a sectional view, along a generic plane, of a body of interest, which has an anomalous region inside, and a plurality of sensor elements applied to the frontier of the body of interest to perform a step of the electromagnetic tomography method of the present invention;
- Figure 2 shows a sectional view of a reference body, which can be virtual or real, having the same shape and dimensions as the body of interest of Figure 1, but devoid of the anomalous region, and the same plurality of sensor elements of Figure 1, used in a further step of the electromagnetic tomography method of the present invention;
- Figure 3 shows a general flow diagram of the tomography method of the present invention;
- Figure 4 shows a graph showing a step of the electromagnetic tomography method of the present invention; and
- Figures 5 and 6 show two further embodiments of the sensor elements for performing the electromagnetic tomography method according to as many embodiments of the invention.

### DESCRIPTION OF EMBODIMENTS

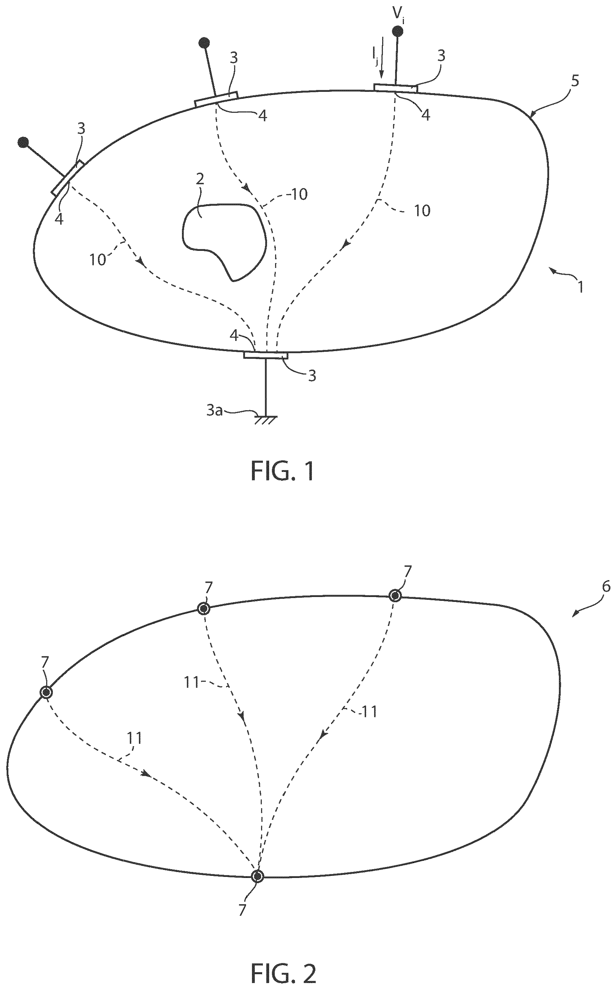

In Figure 1, 1 generically denotes, as a whole, a real body of interest made of one or more materials and electrically or/and magnetically characterised by at least a first physical quantity. In particular, the material or materials of the body of interest 1 has or, respectively, have an internal distribution of a predominantly known physical quantity of interest, i.e. the spatial distribution of the physical quantity of interest is known except for in an anomalous region 2 internal to the body of interest 1, and said first physical quantity derives from the physical quantity of interest and depends on the geometric characteristics of the body of interest 1 and the anomalous region 2. According to a simplifying example, the body of interest 1 is made of a substantially homogeneous material from the point of view of the physical quantity of interest, except for in the anomalous region 2, where a different material is also present.

The extent, position, shape and composition of the anomalous region 2 are unknown and the purpose of the electromagnetic tomography is to identify the position, shape and dimensions of the anomalous region 2 within the body of interest 1. It is assumed that in the anomalous region 2 the physical quantity of interest has a value or values that are unknown and appreciably different (i.e. greater or lesser) from a background distribution of the physical quantity of interest of the remaining volume of the body of interest 1 (i.e. outside the anomalous region 2). In accordance with the above simplifying example, the physical quantity of interest in the anomalous region 2 has values appreciably different from a certain background value, which is characteristic of the remaining volume of the body of interest 1.

With reference again to Figure 1, a plurality of sensor elements 3 are applied to a respective plurality of surface points 4 of an outer surface 5 of the body of interest 1 to apply values of a second physical quantity to the surface points 4 and to measure in the latter corresponding values of a third physical quantity. It can be observed that, although the sensor elements 3 have a certain extension, even small but still non-null, i.e. not attributable to a geometric point, for simplicity of treatment below each portion of the outer surface 5 to which the respective sensor element 3 is applied will be called a surface point, which will be denoted with the same number 4 in the figures.

In the example shown by Figure 1, the body of interest 1 is shown along a plane of cross-section for some sensor elements 3.

The second physical quantity and the third physical quantity are measurable according to known techniques. The third physical quantity is bound to the second physical quantity via a linear relationship that includes the first physical quantity. In particular, the third physical quantity is obtained by a multiplication (matrix per vector) between the first physical quantity and the second physical quantity. In other words, the material or materials of the body of interest 1 define a linear relationship between the second physical quantity and the third physical quantity.

Anticipating and generalizing what will be described in greater detail later in this discussion, since a plurality of sensors 3 are used, the second physical quantity and the third physical quantity will give rise to two respective column vectors, each of which gathers a respective plurality of measurable values, and the first physical quantity will give rise to a square matrix of measurable values that defines the linear relationship between the two column vectors.

In general, the values of the second physical quantity applied to the surface points 4 are stationary or variable depending on periodic waveforms at low frequency, so that the duration of the electromagnetic transients is much less than the minimum time scale of the applied signals. From a physical point of view, this means that the diffusion time of the electromagnetic field is much less than the minimum time scale of the applied signals.

Depending on the electromagnetic characteristics of the material, it is possible to identify a limit value for the time variability of the oscillating waveforms. In particular, being *ε* the electrical permittivity of the material, *σ* the electrical conductivity, *µ* the magnetic permeability and L the linear dimension of the body of interest, it is possible to define two critical time constants *τₑ* and *τₘ* which can be calculated as follows: ${τ}_{e} = ε / σ ,$ ${τ}_{m} = {L}^{2} μσ .$

In order for the duration of the electromagnetic transients to be much less than the minimum time scale of the applied signals, the excitation signal band *ω_{M}* expressed in terms of angular pulsation, must satisfy the following relationship: ${ω}_{M} ≪ min \left\{1/{τ}_{e},1/{τ}_{m}\right\} .$

Figure 2 shows a reference body 6, which is a virtual body or a real body, has the same shape and dimensions as the body of interest 1, and in general is, in the case of a virtual body, numerically modelled (by software) with the same materials as the body of interest 1, or, in the case of a real body, is made of the same materials as the body of interest 1. In particular, the reference body 6 has a known volumetric distribution of the physical quantity of interest, i.e. it corresponds to the body of interest 1, but it is devoid of any anomalous inner portion, such as the anomalous region 2. The reference body 6 has a plurality of frontier points 7 whose positions correspond to those of the surface points 4 of the body of interest 1. In other words, the reference body 6 is made of the same materials as the body of interest 1 present outside the anomalous region 2.

In the particular example shown by Figures 1 and 2, the first physical quantity is electrical resistance R and the physical quantity of interest is the electrical conductivity σ of the material of the body of interest 1. The electrical conductivity σ₂ of the anomalous region 2 is appreciably different from a background electrical conductivity σ₁ of the remaining volume of the body of interest 1. The second physical quantity is electrical current I fed to the electrodes 3 and the third physical quantity is electrical voltage V measured at the electrodes 3, so that the known linear relationship applies: $V = R ⋅ I .$

Given a number M+1 of surface points 4 and thus of frontier points 7, the plurality of sensor elements 3 comprises a number M+1 of electrodes fixed to the respective M+1 surface points 4. Preferably, a number M of electrodes 3 and thus of frontier points 7 are considered active, in the sense that will become clear later, and the M+1-th electrode 3 will be connected to ground, denoted with 3a in Figure 1, i.e. the M+1-th frontier point 7 will be considered at a reference electrical potential (null). In the example shown by Figures 1 and 2, for simplicity M = 3.

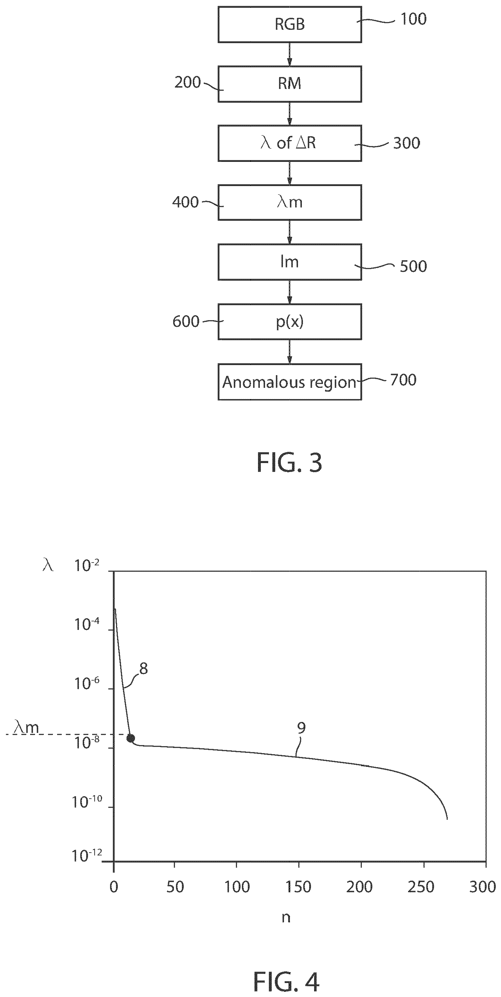

The electromagnetic tomography method of the present invention will be described below with particular reference to the aforesaid example, in which the first physical quantity is electrical resistance R and therefore it is an electrical resistance tomography, and with reference to the general flow diagram shown in Figure 3.

Values of electrical current Iⱼ, where j ∈ {1,...,M}, are applied to respective frontier points 7 of the reference body 6, values of electrical voltage Vᵢ, where i ∈ {1,...,M}, are determined at the same frontier points 7 in terms of potential difference with respect to the M+1-th frontier point 7, and a first matrix of electrical resistance values RBG is determined as a function of the values of electrical current Iⱼ and of the values of electrical voltage Vᵢ (step 100 of Figure 3).

In particular, the values of electrical current Iⱼ are applied one at a time to the respective frontier points 7 and for each value of electrical current Iⱼ a respective plurality of values of electrical voltage Vᵢ at all frontier points 7 is determined and a respective plurality of electrical resistance values of the matrix RBG is calculated. In essence, the plurality of electrical resistance values determined for a certain value of electrical current Iⱼ applied to the respective j-th frontier point 7 corresponds to a column of the matrix RBG. In other words, the generic element of the matrix RBG can be defined as follows: ${\left.{RBG}_{ij}=\frac{{V}_{i}}{{I}_{j}}\right|}_{{I}_{k} = 0 , k \neq j} .$

In other words, the values of electrical current can be represented by a current column vector I, and the values of electrical voltage can be represented by a voltage column vector V which is bound to the current column vector I via matrix RGB according to the following linear vector relationship: $V = RGB ⋅ I .$

Values of electrical current I'ⱼ, where j ∈ {1,...,M}, are fed to respective electrodes 3 fixed to the body of interest 1, values of electrical voltage V'ᵢ, where i ∈ {1,...,M}, are measured at the electrodes 3 in terms of potential difference with respect to the grounded electrode 3, and a second matrix of electrical resistance values RM is determined as a function of the values of electrical current I'ⱼ and of the values of electrical voltage V'ᵢ (step 200 of Figure 3).

In particular, the values of electrical current I'ⱼ are fed one at a time to the respective electrodes 3 and for each value of electrical current I'ⱼ a respective plurality of values of electrical voltage V'ᵢ is measured at all the electrodes 3 and a respective plurality of electrical resistance values of the matrix RM is calculated. In essence, the plurality of electrical resistance values determined for a certain value of electrical current I'ⱼ corresponds to a column of the matrix RM. In other words, the generic element of the matrix RM can be defined as follows: ${\left.{RM}_{ij}=\frac{V {′}_{i}}{I {′}_{j}}\right|}_{I {′}_{k} = 0 , k \neq j} .$

In other words, the values of electrical current can be represented by a current column vector I', and the values of electrical voltage can be represented by a voltage column vector **V'** which is bound to the current column vector **I** via matrix RM according to the following linear vector relationship: $V ′ = RM ⋅ I ′ .$

According to one embodiment, the reference body 6 is virtual, and in particular it is modelled in numerical form and the values of electrical voltage Vᵢ are calculated via a finite element method when the values of electrical current Iⱼ are applied to the frontier points 7. This requires the use of a computer.

According to an alternative embodiment, the reference body 6 is real, and in particular it is a sample whose volumetric distribution of the electrical conductivity σ of the material of which it is made is previously verified with high precision control instruments, for example by means of X-rays. In this case, at the frontier points 7 respective electrodes (not shown) are fixed like to the body of interest 1 to feed the values of electrical current Iⱼ and to measure the values of electrical voltage Vᵢ. Such an embodiment has no advantages from a computational point of view, but it may have advantages in terms of elimination of experimental errors.

A third matrix ΔR is calculated as the difference between the matrices RM and RBG and the eigenvalues of the matrix ΔR are calculated (step 300 of Figure 3). Each of the matrices RM and RBG is a symmetric matrix M x M, and consequently, the matrix ΔR is a symmetric square matrix having a number M of real eigenvalues.

Thus, the eigenvalues λ of the matrix ΔR are calculated and a minimum eigenvalue λm is selected as that lowest absolute eigenvalue that is not significantly corrupted by the noise introduced by the measured values of the electrical voltage V'ᵢ (step 400 of Figure 3).

As is known, by ordering the eigenvalues of the matrix ΔR according to their absolute value in a decreasing manner, a monotonic trend tending exponentially to zero can be highlighted. The selection of the minimum eigenvalue λm therefore provides for ordering the eigenvalues λ of the matrix ΔR by decreasing absolute value and expressing the decreasing trend on a logarithmic scale in order to "linearize" the exponential trend.

Figure 4 shows an example of eigenvalues of the matrix ΔR ordered in descending order along the axis of the abscissas (n) and expressed in logarithmic scale along the axis of the ordinates (λ). The trend of the logarithmic scale eigenvalues has a first section 8 relative to the highest-valued eigenvalues that has a significant and little variable slope, that is, it can be approximated to a straight line, and a second section 9 relative to the lowest eigenvalues consisting of a plateau that begins with a strong change of slope (reduction), due to the noise introduced by the measures that becomes predominant with respect to the eigenvalues that would tend to zero, in the absence of such noise. Therefore, the minimum eigenvalue λm, i.e. the lowest absolute eigenvalue that is not significantly corrupted by noise, is the one preceding the change of slope of the section 9.

The minimum eigenvalue λm is connected with a corresponding eigenvector of the matrix ΔR (step 500 of Figure 3). The eigenvector connected with the minimum eigenvalue λm is a vector of current values and is indicated below with **Im.**

At this point, the spatial distribution of the power density 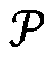 (*x*) in the volume of the reference body 6 is calculated in a known manner when the values of electrical current of the eigenvector **Im** are applied to the frontier points 7 (step 600 of Figure 3). The spatial distribution of power density P(x) is calculated numerically as a function of the background electrical conductivity σ₁ and of the current density arising from the application of the electrical current configuration defined by the eigenvector **Im.**

Ideally, that is, in the absence of measurement errors and using a large M+1 number of electrodes 3 as desired, the spatial distribution of power density 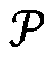 (*x*) in the anomalous region 2 can be made arbitrarily small. In practical applications, taking into account the measurement error and the fact that the number M+1 is finite, the region of the reference body 6 in which the spatial distribution of power density 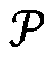 (*x*) is less than an appropriate threshold value PT corresponds to the volume portion of the body of interest 1 that includes the anomalous region 2. Therefore, the anomalous region 2 is identified as that corresponding to the region of the reference body 6 in which the spatial distribution of power density 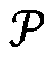 (*x*) is less than the threshold value PT (step 700 of Figure 3).

The threshold value PT must be selected consistently with the noise level introduced by the measured values of electrical voltage V'ᵢ. In particular, the threshold value PT is determined as a function of the minimum eigenvalue λm. More in detail, the threshold value PT is selected so that the integral of the spatial distribution of power density 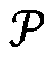 (*x*) extended to all the points of the reference body 6 wherein the spatial distribution of power density 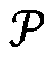 (*x*) is less than or equal to PT, is equal to k·λm, where k is an appropriate constant.

It should be noted that steps 100 and 700 described above are performed by means of a suitably configured computer, starting from the values of electrical current I'ⱼ fed to the electrodes 3 and to the measured values of electrical voltage V'ᵢ. The measured values of electrical voltage V'i are acquired through an analogue-to-digital conversion device of known type integrated or interfaced to the computer. In accordance with the embodiment in which the reference body 6 is a real body, the values of electrical voltage Vᵢ are also measured with the aforesaid analogue-to-digital conversion device.

The physical-mathematical foundation underlying the method of the invention is described below, with particular reference to Figures 1 and 2.

Assuming to find a configuration of values of electrical current I to be applied to the electrodes 3 such as to generate electrical current paths inside the material of the body of interest 1 that do not interact with the anomalous region 2, such as those denoted with 10 in Figure 1, then such an electrical current configuration would generate the same electrical current density paths even in the absence of the anomalous region 2, i.e. inside the reference body 6, denoted with 11 in Figure 2. This implies that the values of electrical voltage V'ᵢ measured on the electrodes 3 applied to the body of interest 1 will be equal to values of electrical voltage Vᵢ calculated at the frontier points 7 of the reference body 6.

To determine the configuration of values of electrical current I to be applied to the electrodes 3, in order to have electrical current density paths that do not interact with the anomalous region 2, the following vector relationships are used: $V = RBG ⋅ I$ $V ′ = RM ⋅ I$ and the constraint V = V' is imposed, from which it follows: $\left(RM-RBG\right) ⋅ I = 0 .$

In other words, the electrical current vector I sought is nothing more than an eigenvector of the difference matrix RM - RBG connected with a null eigenvalue. In practice, by effect of the noise and of the finite number M+1, instead of considering null eigenvalues, sufficiently small eigenvalues are considered. In particular, the last eigenvalue before section 9 is taken into consideration because it is not reasonably significantly corrupted by noise.

The electromagnetic tomography method described above is substantially independent of the excitation pattern of the surface points 4 and of the choice of the mass point that might not be connected with a physical point.

According to a not shown embodiment of the invention, all the M+1 electrodes 3 are active and at the M+1-th electrode 3, instead of being connected to ground, there is applied a value of electrical current of intensity equal to the sum of the values of electrical current applied to the other M electrodes 3 and of opposite sign to said sum.

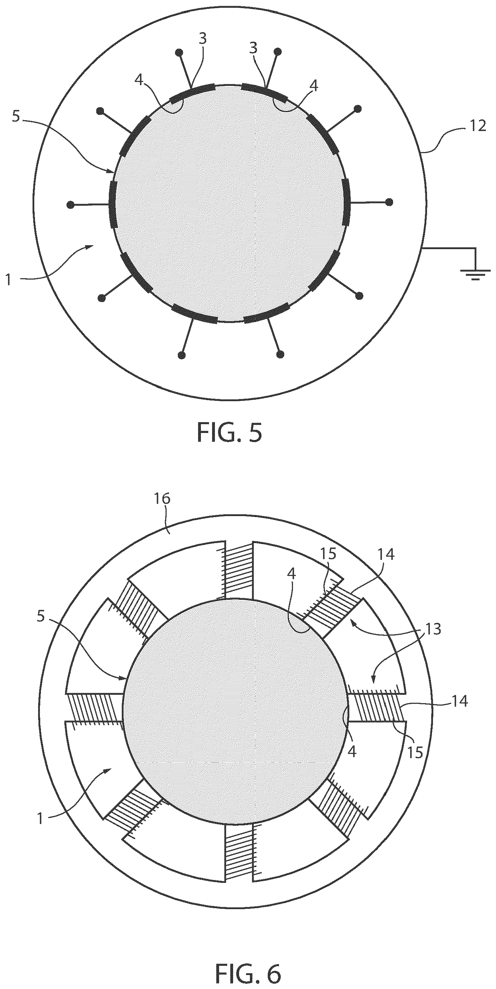

According to a further embodiment of the present invention shown in Figure 5, in which the corresponding elements are denoted with the same numbers and abbreviations as in Figure 1, said physical quantity of interest, whose volumetric distribution in the body of interest 1 is known, is the dielectric permittivity ε, i.e. it is assumed that the dielectric permittivity ε₂ of the anomalous region 2 is appreciably different from a background dielectric permittivity ε₁ of the remaining volume of the body of interest 1, and said first physical quantity is electrical capacity C.

With reference to Figure 5, the electrodes 3 are equal to M, and there is a screen 12, which is external to the body of interest 1 and is connected to ground. Similarly, the reference body 6 has a number equal to M of frontier points 7. The same measurement system described above for the body of interest is applied to the reference body. The second physical quantity is the time derivative of an electrical voltage V variable in time that is applied to the electrodes 3 and the third physical quantity is electrical current I measured at the electrodes 3, so that the following linear relationship applies: $I = C ⋅ \frac{dV}{dt} .$

The embodiment of Figure 5 differs from that of Figure 1 in that waveforms of electrical voltage Vⱼ, where j ∈ {1,...,M}, are applied to respective frontier points 7 of the reference body 6 in terms of potential difference with respect to a point external to the reference body 6, waveforms of electrical current Iᵢ are determined at the frontier points 7 of the reference body 6, waveforms of electrical voltage V'ⱼ, where j ∈ {1,...,M}, are applied to the respective electrodes 3 fixed to the body of interest 1 in terms of potential difference with respect to the screen 12, and waveforms of electrical current I'ᵢ are measured at the electrodes 3 and, instead of the three matrices RBG, RM and ΔR, three matrices of electric capacitance values CBG, CM and ΔC are determined in a similar way, wherein the elements of the matrices CBG and CM are calculated as follows: ${\left.{CBG}_{ij}=\frac{{I}_{i}}{\frac{{dV}_{j}}{dt}}\right|}_{{V}_{k} = 0 , k \neq j} ,$ ${\left.{CM}_{ij}=\frac{I {′}_{i}}{\frac{dV {′}_{j}}{dt}}\right|}_{V {′}_{k} = 0 , k \neq j} ,$ and the third matrix ΔC is calculated as the difference between the matrices CM and CBG.

In other words, the matrices CBG and CM define, between two column vectors of derivative of voltage **DV** and of current I and, respectively, between two further column vectors of derivative of voltage **DV'** and of current **I',** the following linear vector relationships: $I = CBG ⋅ DV ,$ $I ′ = CM ⋅ DV ′ .$

The derivatives of the waveforms of electrical voltage Vⱼ and V'ⱼ that make up the voltage derivative vectors **DV** and **DV'** are calculated numerically or analytically starting from the voltage waveforms themselves that are known as input variables. Obviously, the application of an electrical voltage waveform to a frontier point 7 or electrode 3 corresponds de facto to the application at the same frontier point 7 or electrode 3 of a corresponding time derivative waveform of this electrical voltage (said second physical quantity).

Although the voltage derivative column vectors **DV** and **DV'** and the current vectors **I** and **I'** are waveform vectors having a certain time trend, the elements of the matrices CBG and CM are constant over time. The eigenvector connected with the minimum eigenvalue λm, which is defined as the minimum eigenvalue of a matrix ΔC calculated as the difference between the matrices CM and CBG not significantly corrupted by noise, is a vector of constant values of first derivative of voltage and is therefore denoted with **DVm.**

Furthermore, the embodiment of Figure 5 differs from that of Figure 1 in that the spatial distribution of the electrical energy density *ε*(*x*) in the volume of the reference body 6 is calculated when the waveforms of electrical voltage corresponding to the eigenvector **DVm** are applied to the frontier points 7. The vector of the waveforms of electrical voltage applied to the frontier points 7, hereinafter referred to as **Vm,** can be expressed as the product between the eigenvector **DVm** and the variable time t, i.e. the electrical voltages applied to the frontier points 7 have linearly increasing waveforms over time. The spatial distribution of electrical energy density *ε*(*x*) is, therefore, a quadratic function of time, namely: $E \left(x, t\right) = {\left(t/{t}_{0}\right)}^{2} ⋅ {E}_{0} \left(x\right) ,$ where *ε*₀(*x*) is the spatial distribution of electrical energy density corresponding to an arbitrary time instant *t*₀, hereinafter also called the spatial distribution of reference electrical energy density.

The spatial distribution of reference electrical energy density *ε*₀(*x*) is calculated numerically as a function of the background dielectric permittivity ε₁ and of the electric field that arises from the application of the vector of the waveforms of electrical voltage **Vm** at the time instant t_{0,}, i.e. t₀·**DVm**.

The anomalous region 2 is identified as that corresponding to the region of the reference body 6 in which the spatial distribution of reference electrical energy density *ε*₀(*x*) is less than a threshold value ET. The threshold value ET is selected so that the integral of the spatial distribution of reference electrical energy density *ε*₀(*x*) extended to all points of the reference body 6 wherein this reference distribution *ε*₀(*x*) is less than or equal to ET, is equal to k·λm, where k is an appropriate constant.

In essence, the embodiment described above with reference to Figure 5 realizes an electrical capacitance tomography.

According to a further embodiment of the present invention shown in Figure 6, in which the corresponding elements are denoted with the same numbers and abbreviations as in Figure 1, said physical quantity of interest, whose volumetric distribution in the body of interest 1 is known, is magnetic permeability µ, i.e. it is assumed that the magnetic permeability µ₂ of the anomalous region 2 is appreciably different from a background magnetic permeability µ₁ of the remaining volume of the body of interest 1, and said first physical quantity is inductance L.

With reference to Figure 6, the sensor elements applied to the surface points 4 are equal to M and comprise respective magnetic poles 13. In particular, each magnetic pole 13 is of the electromagnetic type and comprises a ferromagnetic core 14, which has an end facing the respective surface point 4, and a coil 15 wound on the ferromagnetic core. The ferromagnetic cores 14 project from a body 16 of ferromagnetic material adapted to close a magnetic circuit. Similarly, the reference body 6 has a number equal to M of frontier points 7 in front of which respective magnetic poles are arranged. The second physical quantity is the time derivative of an electrical current I variable in time which is injected into the coils 15 and the third physical quantity is electrical voltage that is measured at the ends of the coils 15 and is produced by magnetic induction by the electrical current I variable in time, so that the linear relationship applies: $V = L ⋅ \frac{dI}{dt} .$

The embodiment of Figure 6 differs from that of Figure 1 in that waveforms of electrical current Iⱼ, where j ∈ {1,...,M}, are fed to the magnetic poles of the respective frontier points 7 of the reference body 6, i and waveforms of electrical voltage Vᵢ are determined at the frontier points 7, waveform of electrical current I'ⱼ, where j ∈ {1,...,M}, are fed to the coils 15 of the magnetic poles 13 facing the body of interest 1, waveforms of electrical voltage V'ᵢ are measured at the magnetic poles 13, and in particular are measured at the ends of the coils 15, and, instead of the three matrices RBG, RM and ΔR, three inductance value matrices LBG, LM and ΔL are determined, in a similar way, wherein the elements of the matrices LBG and LM are calculated as follows: ${\left.{LBG}_{ij}=\frac{{V}_{i}}{\frac{{dI}_{j}}{dt}}\right|}_{{I}_{k} = 0 , k \neq j} ,$ ${\left.{LM}_{ij}=\frac{V {′}_{i}}{\frac{dI {′}_{j}}{dt}}\right|}_{I {′}_{k} = 0 , k \neq j} ,$ and the third matrix ΔL is calculated as the difference between the matrices LM and LBG.

In other words, the matrices LBG and LM define, between two column vectors of derivative of current **DI** and of voltage **V** and, respectively, between two further column vectors of derivative of current **DI'** and of voltage **V'** the following linear vector relationships: $V = LBG ⋅ DI ,$ $V ′ = LM ⋅ DI ′ .$

The derivatives of the waveforms of electrical voltage Iⱼ and I'ⱼ that make up the current derivative vectors **DI** and **DI'** are calculated numerically or analytically starting from the current waveforms themselves that are known as input variables. Obviously, the application of an electrical current waveform to a frontier point 7 or electrode 3 corresponds de facto to the application at the same frontier point 7 or electrode 3 of a corresponding time derivative waveform of that electrical current (said second physical quantity).

Although the current derivative column vectors **DI** and **DI'** and the voltage vectors **V** and **V'** are waveform vectors having a certain time trend, the elements of the matrices LBG and LM are constant over time. Also in this case, the eigenvector connected with the minimum eigenvalue λm, which is defined as the minimum eigenvalue of a matrix ΔL calculated as the difference between the matrices LM and LBG not significantly corrupted by noise, is a vector of constant values of first derivative of current and is therefore denoted with **DIm.**

Furthermore, the embodiment of Figure 6 differs from that of Figure 1 in that the spatial distribution of the magnetic energy density 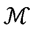 (*x*) in the volume of the reference body 6 is calculated when the corresponding values of electrical current of the eigenvector **DIm** are applied to the frontier points 7. The vector of the waveforms of electrical currents applied to the frontier points 7, hereinafter referred to as **Im,** can be expressed as the product between the eigenvector **DIm** and the variable time t, i.e. the electrical currents applied to the frontier points 7 have waveforms increasing linearly over time. The spatial distribution of magnetic energy density (*x*) is, therefore, a quadratic function of time, namely: $M \left(x, t\right) = {\left(t/{t}_{0}\right)}^{2} ⋅ {M}_{0} \left(x\right) ,$ where 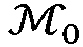 (*x*) is the spatial distribution of magnetic energy density corresponding to an arbitrary time instant *t*₀, hereinafter also called spatial distribution of reference magnetic energy density.

The spatial distribution of the reference magnetic energy density 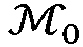 (*x*) is calculated numerically as a function of the background magnetic permeability µ1 and of the magnetic field that arises from the application of the vector of the waveforms of electrical current **Im** to the time instant t₀, i.e. t₀·**DIm**.

The anomalous region 2 is identified as that corresponding to the region of the reference body 6 in which the spatial distribution of reference magnetic energy density 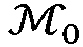 (*x*) is less than a threshold value MT. The threshold value MT is selected so that the integral of the spatial distribution of reference magnetic energy density 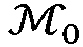 (*x*) extended to all points of the reference body 6 wherein this reference distribution 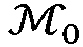 (*x*) is less than or equal to ET, is equal to k·λm, where k is an appropriate constant.

In essence, the embodiment described above with reference to Figure 6 performs magnetic inductance tomography.

The electromagnetic tomography method in the various embodiments described above is substantially independent of the mode of excitation of the frontier points 7 of the reference body 6 of the surface points 4 of the body of interest 1. In the various embodiments described above, in general, the second physical quantity means that physical quantity used to excite the reference body 6 and the body of interest 1 and the third physical quantity means that physical quantity that is measured.

According to further embodiments of the invention not shown, the second physical quantity and the third physical quantity are exchanged with respect to the embodiments described with reference to Figures 1-6, or replaced by suitable linear combinations, also of elements of the vectors of the second and third physical quantity.

For example, with particular reference to an embodiment derived from that described with reference to Figures 1-2, voltage values Vⱼ and, respectively, V'ⱼ are applied to the frontier points 7 of the reference body 6 and to the electrodes 3 on the body of interest 1 and then corresponding current values Iᵢ and, respectively, I'ᵢ are measured.

With particular reference to an embodiment derived instead from that shown in Figure 5, waveforms of electrical current Iⱼ and, respectively, I'ⱼ are applied to the frontier points 7 of the reference body 6 and to the electrodes 3 on the body of interest 1 and then corresponding waveforms of electrical voltage Vᵢ and, respectively, V'ᵢ are measured.

With particular reference to an embodiment derived instead from that shown in Figure 6, waveforms of electrical voltage Vⱼ and, respectively, V'ⱼ are applied to the frontier points 7 of the reference body 6 and to the electrodes 3 on the body of interest 1, and then corresponding waveforms of electrical current Iᵢ and, respectively, I'ᵢ are measured.

According to further embodiments of the invention not shown, both the second physical quantity and the third physical quantity are used to excite and, in a complementary manner, measure the reference body 6 and the body of interest 1, i.e. a part of frontier points 7 and of corresponding surface points 4 are fed in terms of second physical quantity and the remaining part in terms of third physical quantity, and in the fed points in terms of second physical quantity the third physical quantity is measured and in the fed points in terms of third physical quantity the second physical quantity is measured.

The main advantage of the electromagnetic tomography method described above in various embodiments is the drastic reduction of the overall computational cost, as will be explained in detail below, assuming to adopt a numerical method for the solution of a direct problem, which is attributable to the solution of a system of linear equations, and the Gauss's method for the solution of the corresponding system of linear equations, in which the system is solved in two steps: a first step of factorization LU of the matrix that defines the system of equations and a second step of "back-substitution" for the rapid resolution of two linear systems characterized by lower L (Lower) and upper U (Upper) triangular matrices.

The computation cost of the determination step of the first matrix RBG, CBG, LBG depends on the nature of the reference body 6, i.e. whether the latter is a virtual body or a real body. These two different embodiments relative to the reference body 6 have been described above with specific reference to the determination of the matrix RBG but are also applicable *mutatis mutandis* to the matrices CBG and LBG.

In the case of a virtual reference body 6, referring for example to a method based on finite elements, it is possible to demonstrate that the computational cost of the determination step of the first matrix RBG, CBG, LBG, can be estimated as follows: $CompBG = \frac{1}{3} {N}^{3} + M ⋅ O \left({N}^{2}\right) ,$ wherein N is the number of nodes of the mesh due to the discretization of the volume of the reference body 6, N³/3 is the cost for the factorization LU of the matrix representative of the linear system coming from the numerical method (called *stiffness matrix*), M is the number of the active sensor elements and *M ·* 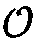 (*N*²) is the computational cost for the calculation of the values of the third physical quantity on the frontier points 7, which derives from the numerical solution of M systems of linear equations via back-substitution, upon variation of the applied excitation, once the stiffness matrix factorization is known.

In the case, however, of real reference body 6, the first matrix RGB, CBG, LBG is determined experimentally through measurements and therefore the cost of determining this matrix is bound to the measurement protocol used of the first matrix RBG, CBG, LBG. However, the step of determining the spatial distribution of power density 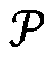 (*x*) or of electrical energy *ε*(*x*) or of magnetic energy 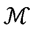 (*x*) requires numerically solving the direct problem in the presence of the virtual reference body and of the eigenvector **Im, DVm, DIm.** The solution to this direct problem provides for stiffness matrix factorization connected with the first matrix RBG, CBG, LBG which has a computational cost that can be estimated as follows: $CompBG = \frac{1}{3} {N}^{3} .$

Therefore, in the case of real reference body 6, only the cost linked to the stiffness matrix factorization is taken into account, while the cost of the experimental determination of the first matrix RBG, CGB, LGB is linked to the measurement protocol used.

It is also possible to demonstrate that the computational cost of the processing steps downstream of the measurements of the third physical quantity on the body of interest 1 can be estimated as follows: $CompM = 2 ⋅ {N}^{2} + O \left({M}^{3}\right) ,$ wherein 2·N² is the contribution due to the calculation of the spatial distribution of the power or energy density and 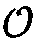 (*M*³) is the contribution bound to the calculation of the eigenvalues λ and to the selection of the minimum eigenvalue λm of the third matrix ΔR, ΔC, ΔL, having dimension M-x-M. The identification of the eigenvalue connected with the minimum eigenvalue λm and of the anomalous region 2 through the evaluation of the distribution of the power or energy density are negligible from the point of view of the computational cost.

The step of determining the second matrix RM, CM, LM does not have a real computational cost, but it simply requires a certain time for execution that depends on the protocol used for the measurement of the third physical quantity.

The first matrix RBG, CBG, LBG must be calculated only once on the basis of the shape of the body of interest 1, of the position of the surface points 4 of the background value σ₁, ε₁, µ₁ of the physical quantity of interest (electrical conductivity, dielectric permittivity, magnetic permeability), i.e. the computational cost relative to the determination of the stiffness matrix factorization is "spent" only once before making the measurements of the third physical quantity. In other words, the stiffness matrix, connected with the configuration of the reference body 6, is factored once and then reused in the subsequent processing steps in which the solution of the direct problem appears in the presence of the relative background physical quantity of interest (electrical conductivity, dielectric permittivity, magnetic permeability). The computational advantage is that factoring has a cost equal to N³/3 while back-substituting has a cost equal to 2·N². Therefore, having the factorization of the first stiffness matrix available, the cost of the solution to the direct problem drops to a quadratic type complexity.

The overall computational cost is contributed by the calculation of the spatial distribution of the power or energy density, whose computational cost is equal to 2·N², and the calculation of the eigenvalues λ and of the minimum eigenvalue λm of the third matrix ΔR, ΔC, ΔL, which has a MxM dimension and therefore requires a computational cost equal to 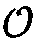 (*M*³). The identification of the eigenvector connected with the minimum eigenvalue λm and of the anomalous region 2 by evaluating the spatial distribution of power or energy density are negligible in terms of computational cost.

In summary, in the case of virtual reference body 6, the computational cost spent upstream of the measurements made on body of interest 1 can be estimated as follows: $CompBG = \frac{1}{3} {N}^{3} + M ⋅ O \left({N}^{2}\right) ;$ while in the case of real reference body 6, the aforesaid computational cost can be estimated as follows: $CompBG = \frac{1}{3} {N}^{3} .$

For both of the aforesaid embodiments, the computational cost required for operations downstream of the measurements on the body of interest 1 can be estimated as follows: $CompM = 2 ⋅ {N}^{2} + O \left({M}^{3}\right) .$

Considering that the computational cost of a generic iterative method of a known type, previously denoted with *Comp,* can be estimated through the relationship $Comp = \frac{K}{3} {N}^{3} + M ⋅ K ⋅ O \left({N}^{2}\right) ,$ that the computational cost relative to the stiffness matrix factorization, and therefore the computational cost *CompBG,* is spent only once before making the measurements of the third physical quantity, and that N is much greater than M, since the number N of the nodes of the discretization mesh can exceed one thousand and the number M of sensor elements applied to the body of interest 1 cannot be high, it can be observed that the invention allows to pass from the computational cost proportional to N³ of the iterative methods to a computational cost proportional to N². In other words, since the stiffness matrix factorization can be precalculated without having to interact with the body of interest 1, the computational cost spent downstream of the measurements on the body of interest is of the order of N².

In iterative methods it is not possible to operate by pre-calculating the stiffness matrix, since downstream of the measurements on the body of interest 1 it is necessary, with each iteration, to solve a linear problem characterized by a different physical quantity of interest with respect to the previous problem, therefore by a different stiffness matrix, thus having to proceed with a new factorization.

If one then operates a number K of iterations proportional to the number of nodes N, then the computation cost of the iterative methods would be proportional to N⁴ and the advantage of the invention would be even greater.

## Claims

1. An electromagnetic tomography method for detecting anomalies in a body of interest (1), the body of interest (1) being electrically or/and magnetically **characterized by** at least a first physical quantity (R; C; L), which is derived from a physical quantity of interest (σ; ε; µ) and binds a second physical quantity (I; dV/dt; dI/dt) to a third physical quantity (V; I; V) via a linear relationship, the method comprising the following steps performed by a computer:
- for a real or virtual reference body (6) having the same shape and dimensions as the body of interest (1) and having a known volumetric distribution of the physical quantity of interest (σ; ε; µ), determining (100) a first matrix (RBG; CBG; LBG) of values of the first physical quantity (R C; L) as a function of values or waveforms of the second physical quantity (Iⱼ; dVⱼ/dt; dIⱼ/dt) applied to respective frontier points (7) of the reference body (6) and as a function of values or waveforms of the third physical quantity (Vᵢ; Iᵢ; Vᵢ) measured or calculated at the frontier points (7);
- determining (200) a second matrix (RM; CM; LM) of values of the first physical quantity (R; C; L) as a function of further values or waveforms of the second physical quantity (I'ⱼ; dV'ⱼ/dt; dI'ⱼ/dt) fed to respective sensor elements (3; 13) applied to respective surface points (4) of the body of interest (1) corresponding to the frontier points (7), and as a function of further values or waveforms of the third physical quantity (V'ᵢ; I'ᵢ; V'ᵢ) measured by the sensor elements (3; 13);
- determining (300) the eigenvalues (λ) of a third matrix (ΔR; ΔC; ΔL) calculated as the difference between the second matrix (RM; CM; LM) and the first matrix (RBG; CBG; LBG); and
- selecting (400) a minimum eigenvalue (λm) among said eigenvalues (λ) as that lowest-valued eigenvalue that is not significantly corrupted by the noise introduced by the measured values of the third physical quantity (V'ᵢ; I'ᵢ; V'ᵢ);
the method **being characterized by** comprising the following further steps performed by the computer:
- extracting (500) the eigenvector **(Im; DVm; DIm)** of the third matrix (ΔR; ΔC; ΔL) corresponding to the lowest eigenvalue (λm);
- calculating (600) the spatial distribution of power or energy density ( 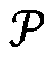 (*x*)); *ε*₀(*x*); 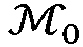 (*x*)) in the volume of the reference body (6) when the values of the eigenvector **(Im; DVm; DIm)** are applied to the frontier points (7); and
- identifying (700) at least one anomalous region (2) of the body of interest (1) as the one corresponding to a region of the reference body (6) in which the power or energy density ( 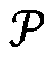 (*x*)); *ε*₀(*x*); 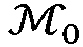 (*x*)) is less than a threshold value (PT; ET; MT).

2. The method according to claim 1, wherein determining (100) a first matrix (RBG; CBG; LBG) of values of the first physical quantity (R; C; L) comprises:
- applying the values or waveforms of the second physical quantity (Iⱼ; dVⱼ/dt; dIⱼ/dt) one at a time to the respective frontier points (7); and
- for each value or waveform of the second physical quantity (Iⱼ; dVⱼ/dt; dIⱼ/dt), measuring or calculating a respective plurality of values or waveforms of the third physical quantity (Vᵢ; Iᵢ; Vᵢ) at all respective frontier points (7) and calculating a respective plurality of values of the first matrix (RBG; CBG; LBG), each as a function of the value or waveform of the second physical quantity (Iⱼ; dVⱼ/dt; dIⱼ/dt) and a respective value or waveform of the plurality of values or waveforms of the third physical quantity (Vᵢ; Iᵢ; Vᵢ).

3. The method according to claim 1 or 2, wherein determining (200) a second matrix (RM; CM; LM) of values of the first physical quantity (R; C; L) comprises:
- feeding the further values or waveforms of the second physical quantity (I'ⱼ; dV'ⱼ/dt; dI'ⱼ/dt) one at a time to respective sensor elements (3; 13); and
- for each further value or waveform of the second physical quantity (I'ⱼ; dV'ⱼ/dt; dI'ⱼ/dt), measuring a respective plurality of further values or waveforms of the third physical quantity (V'ᵢ; I'ᵢ; V'ᵢ) via all sensor elements (3; 13) and calculating a respective plurality of values of the second matrix (RM; CM; LM), each according to the further value or waveform of the second physical quantity (I'ⱼ; dV'ⱼ/dt; dI'ⱼ/dt) and a respective value or waveform of the plurality of further values or waveforms of the third physical quantity (V'ᵢ; I'ᵢ; V'ᵢ).

4. The method according to any one of claims 1 to 3, wherein selecting (400) a minimum eigenvalue (λm) from said eigenvalues (λ) comprises:
- ordering said eigenvalues (λ) in descending order and expressing the descending trend in a logarithmic scale;
- selecting the lowest eigenvalue preceding a change of slope of the decreasing trend on the logarithmic scale.

5. The method according to any one of claims 1 to 4, wherein said first physical quantity is electrical resistance (R), the physical quantity of interest is electrical conductivity (σ), the second physical quantity is electrical current (I), the third physical quantity is electrical voltage (V) and the sensing elements are electrodes (3) in contact with the respective surface points (4).

6. The method according to any one of claims 1 to 4, wherein said first physical quantity is electrical capacitance (C), the physical quantity of interest is dielectric permittivity (ε), the second physical quantity is the time derivative of electrical voltage (dV/dt), the third physical quantity is electrical current (I) and the sensor elements are electrodes (3) in contact with respective surface points (4).

7. The method according to any one of claims 1 to 4, wherein said first physical quantity is electrical capacitance (C), said physical quantity of interest is magnetic permeability (µ), said second physical quantity is the time derivative of electrical current (dI/dt), said third physical quantity is electrical voltage (V) and said sensor elements are magnetic poles (13) facing respective surface points (4).

## Patentansprüche

1. Elektromagnetisches Tomografieverfahren zum Erkennen von Anomalien in einem Körper von Interesse (1), wobei der Körper von Interesse (1) durch mindestens eine erste physikalische Größe (R; C; L) elektrisch oder/und magnetisch charakterisiert ist, die aus einer physikalischen Größe von Interesse (σ; ε; µ) abgeleitet wird und eine zweite physikalische Größe (I; dV/dt; dI/dt) über eine lineare Beziehung mit einer dritten physikalischen Größe (V; I; V) verknüpft, wobei das Verfahren die folgenden, von einem Computer ausgeführten Schritte umfasst:
- für einen realen oder virtuellen Referenzkörper (6), der dieselbe Form und dieselben Abmessungen wie der Körper von Interesse (1) aufweist und eine bekannte Volumenverteilung der physikalischen Größe von Interesse (σ; ε; µ) besitzt, Ermitteln (100) einer ersten Matrix (RBG; CBG; LBG) aus Werten der ersten physikalischen Größe (R; C; L) als Funktion von Werten oder Wellenformen der zweiten physikalischen Größe (Iⱼ; dVⱼ/dt; dIⱼ/dt), die an jeweiligen Randpunkten (7) des Referenzkörpers (6) angelegt werden, sowie als Funktion von Werten oder Wellenformen der dritten physikalischen Größe (Vᵢ; Iᵢ; Vᵢ), die an den Randpunkten (7) gemessen oder berechnet werden;
- Ermitteln (200) einer zweiten Matrix (RM; CM; LM) von Werten der ersten physikalischen Größe (R; C; L) als Funktion weiterer Werte oder Wellenformen der zweiten physikalischen Größe (I'ⱼ; dV'ⱼ/dt; dI'ⱼ/dt), die den jeweiligen Sensorelementen (3; 13) zugeführt werden, die an den jeweiligen Oberflächenpunkten (4) des Körpers von Interesse (1) angebracht sind, die den Grenzpunkten (7) entsprechen, sowie als Funktion weiterer Werte oder Wellenformen der dritten physikalischen Größe (V'ᵢ; I'ᵢ; V'ᵢ), die von den Sensorelementen (3; 13) gemessen werden;
- Ermitteln (300) der Eigenwerte (λ) einer dritten Matrix (ΔR; ΔC; ΔL), die als Differenz zwischen der zweiten Matrix (RM; CM; LM) und der ersten Matrix (RBG; CBG; LBG) berechnet wird; und
- Auswählen (400) eines minimalen Eigenwerts (λm) aus den Eigenwerten (λ) als den Eigenwert mit dem niedrigsten Wert, der nicht wesentlich durch das Rauschen verfälscht ist, das durch die Messwerte der dritten physikalischen Größe (V'ᵢ; I'ᵢ; V'ᵢ) verursacht wird;
wobei das Verfahren **dadurch gekennzeichnet ist, dass** es die folgenden weiteren, vom Computer ausgeführten Schritte umfasst:
- Extrahieren (500) des Eigenvektors (Im; DVm; DIm) der dritten Matrix (ΔR; ΔC; ΔL), der dem kleinsten Eigenwert (λm) entspricht;
- Berechnen (600) der räumlichen Verteilung der Leistungs- oder Energiedichte (*P*(*x*)); ε₀(*x*); *M*₀(*x*)) im Volumen des Referenzkörpers (6), wenn die Werte des Eigenvektors (Im; DVm; DIm) auf die Randpunkte (7) angewendet werden; und
- Identifizieren (700) mindestens eines anomalen Bereichs (2) des Körpers von Interesse (1) als denjenigen, der einem Bereich des Referenzkörpers (6) entspricht, in dem die Leistungs- oder Energiedichte (*P*(*x*)); ε₀(*x*); *M*₀(*x*)) kleiner ist als ein Schwellenwert (PT; ET; MT).

2. Verfahren nach Anspruch 1, wobei das Ermitteln (100) einer ersten Matrix (RBG; CBG; LBG) von Werten der ersten physikalischen Größe (R; C; L) Folgendes umfasst:
Anwenden der Werte oder Wellenformen der zweiten physikalischen Größe (Iⱼ; dVⱼ/dt; dIⱼ/dt) nacheinander auf die jeweiligen Randpunkte (7); und
- für jeden Wert oder jede Wellenform der zweiten physikalischen Größe (Iⱼ; dVⱼ/dt; dIⱼ/dt), Messen oder Berechnen einer entsprechenden Vielzahl von Werten oder Wellenformen der dritten physikalischen Größe (Vᵢ; Iᵢ; Vᵢ) an allen jeweiligen Grenzpunkten (7) und Berechnen einer entsprechenden Vielzahl von Werten der ersten Matrix (RBG; CBG; LBG), jeweils als Funktion des Wertes oder der Wellenform der zweiten physikalischen Größe (Iⱼ; dVⱼ/dt; dIⱼ/dt) und eines jeweiligen Wertes oder einer jeweiligen Wellenform aus der Vielzahl von Werten oder Wellenformen der dritten physikalischen Größe(Vᵢ; Iᵢ; Vᵢ)

3. Verfahren nach Anspruch 1 oder 2, wobei das Ermitteln (200) einer zweiten Matrix (RM; CM; LM) von Werten der ersten physikalischen Größe (R; C; L) Folgendes umfasst:
- Zuführen der weiteren Werte oder Wellenformen der zweiten physikalischen Größe (I'ⱼ; dV'ⱼ/dt; dI'ⱼ/dt) nacheinander zu den jeweiligen Sensorelementen (3; 13); und
- für jeden weiteren Wert oder jede weitere Wellenform der zweiten physikalischen Größe (I'ⱼ; dV'ⱼ/dt; dI'ⱼ/dt), Messen einer jeweiligen Vielzahl weiterer Werte oder Wellenformen der dritten physikalischen Größe (V'ᵢ; I'ᵢ; V'ᵢ) über alle Sensorelemente (3; 13) und Berechnen einer jeweiligen Vielzahl von Werten der zweiten Matrix (RM; CM; LM), jeweils entsprechend dem weiteren Wert oder der weiteren Wellenform der zweiten physikalischen Größe (I'ⱼ; dV'ⱼ/dt; dI'ⱼ/dt) und einem jeweiligen Wert oder einer jeweiligen Wellenform aus der Vielzahl weiterer Werte oder Wellenformen der dritten physikalischen Größe (V'ᵢ; I'ᵢ; V'ᵢ).

4. Verfahren nach einem der Ansprüche 1 bis 3, wobei das Auswählen (400) eines minimalen Eigenwerts (λm) aus den Eigenwerten (λ) Folgendes umfasst:
- Ordnen der Eigenwerte (λ) in absteigender Reihenfolge und Darstellen des absteigenden Trends auf einer logarithmischen Skala;
- Auswählen des kleinsten Eigenwerts, der einer Änderung der Steigung des abfallenden Trends auf der logarithmischen Skala vorausgeht.

5. Verfahren nach einem der Ansprüche 1 bis 4, wobei die erste physikalische Größe der elektrische Widerstand (R) ist, die physikalische Größe von Interesse die elektrische Leitfähigkeit (σ) ist, die zweite physikalische Größe der elektrische Strom (I) ist, die dritte physikalische Größe die elektrische Spannung (V) ist und die Sensorelemente Elektroden (3) sind, die mit den jeweiligen Oberflächenpunkten (4) in Kontakt stehen.

6. Verfahren nach einem der Ansprüche 1 bis 4, wobei die erste physikalische Größe die elektrische Kapazität (C) ist, die physikalische Größe von Interesse die dielektrische Permittivität (ε) ist, die zweite physikalische Größe die zeitliche Ableitung der elektrischen Spannung (dV/dt) ist, die dritte physikalische Größe der elektrische Strom (I) ist und die Sensorelemente Elektroden (3) sind, die mit jeweiligen Oberflächenpunkten (4) in Kontakt stehen.

7. Verfahren nach einem der Ansprüche 1 bis 4, wobei die erste physikalische Größe die elektrische Kapazität (C) ist, die physikalische Größe von Interesse die magnetische Permeabilität (µ) ist, die zweite physikalische Größe die zeitliche Ableitung des elektrischen Stroms (dI/dt) ist, die dritte physikalische Größe die elektrische Spannung (V) ist und die Sensorelemente Magnetpole (13) sind, die jeweiligen Oberflächenpunkten (4) zugewandt sind.

## Revendications

1. Méthode de tomographie électromagnétique pour détecter des anomalies dans un corps concerné (1), le corps concerné (1) étant caractérisé électriquement et/ou magnétiquement par au moins une première grandeur physique (R ; C ; L), qui est dérivée d'une grandeur physique d'intérêt (σ ; ε ; µ) et qui lie une deuxième grandeur physique (I ; dV/dt ; dI/dt) à une troisième grandeur physique (V ; I ; V) par le biais d'une relation linéaire, la méthode comprenant les étapes suivantes exécutées par un ordinateur :
- pour un corps de référence (6) réel ou virtuel présentant la même forme et les mêmes dimensions que le corps concerné (1) et ayant une distribution volumétrique connue de la grandeur physique d'intérêt (σ ; ε ; µ), la détermination (100) d'une première matrice (RBG ; CBG ; LBG) des valeurs de la première grandeur physique (R ; C ; L) en fonction de valeurs ou de formes d'onde de la deuxième grandeur physique (Iⱼ ; dVⱼ/dt ; dIⱼ/dt) appliquées à des points de frontière (7) respectifs du corps de référence (6) et en fonction de valeurs ou de formes d'onde de la troisième grandeur physique (Vᵢ; Iᵢ; Vᵢ) mesurées ou calculées aux points de frontière (7) ;
- la détermination (200) d'une deuxième matrice (RM ; CM ; LM) de valeurs de la première grandeur physique (R ; C ; L) en fonction de valeurs ou de formes d'onde supplémentaires de la deuxième grandeur physique (I'ⱼ; dV'ⱼ/dt; dI'ⱼ/dt) alimentées aux éléments capteurs respectifs (3 ; 13) appliqués sur des points de surface (4) respectifs du corps concerné (1) correspondant aux points de frontière (7), et en fonction de valeurs ou de formes d'onde supplémentaires de la troisième grandeur physique (V'ᵢ; I'ᵢ; V'ᵢ) mesurées par les éléments capteurs (3 ; 13) ;
- la détermination (300) des valeurs propres (λ) d'une troisième matrice (ΔR ; ΔC ; ΔL) calculée comme étant la différence entre la deuxième matrice (RM ; CM ; LM) et la première matrice (RBG ; CBG ; LBG) ; et
- la sélection (400) d'une valeur propre minimale (λm) parmi lesdites valeurs propres (λ), comme étant la valeur propre la plus faible qui ne soit pas significativement faussée par le bruit introduit par les valeurs mesurées de la troisième grandeur physique (V'ᵢ; I'ᵢ; V'ᵢ) ;
la méthode étant **caractérisée en ce qu'**elle comprend les étapes supplémentaires suivantes, exécutées par l'ordinateur :
- l'extraction (500) du vecteur propre (Im ; DVm ; DIm) de la troisième matrice (ΔR ; ΔC ; ΔL) correspondant à la valeur propre la plus faible (λm) ;
- le calcul (600) de la répartition spatiale de la densité de puissance ou d'énergie (*P*(*x*)); ε₀(*x*); *M*₀(*x*)) dans le volume du corps de référence (6) lorsque les valeurs du vecteur propre (Im ; DVm ; DIm) sont appliquées aux points de frontière (7) ; et
- l'identification (700) d'au moins une région anormale (2) du corps concerné (1) comme étant celle qui correspond à une région du corps de référence (6) dans laquelle la densité de puissance ou d'énergie (*P*(*x*)); ε₀(*x*); *M*₀(*x*)) est inférieure à une valeur seuil (PT ; ET ; MT).

2. Méthode selon la revendication 1, la détermination (100) d'une première matrice (RBG ; CBG ; LBG) de valeurs de la première grandeur physique (R ; C ; L) comprenant :
l'application des valeurs ou des formes d'onde de la deuxième grandeur physique (Iⱼ; dVⱼ/dt; dIⱼ/dt) une par une aux points de frontière respectifs (7) ; et
- pour chaque valeur ou forme d'onde de la deuxième grandeur physique (Iⱼ ; dVⱼ/dt ; dIⱼ/dt), la mesure ou le calcul d'une pluralité respective de valeurs ou de formes d'onde de la troisième grandeur physique (Vᵢ ; Iᵢ ; Vᵢ) à tous les points de frontière (7) respectifs et le calcul d'une pluralité respective de valeurs de la première matrice (RBG ; CBG ; LBG), chacune en fonction de la valeur ou de la forme d'onde de la deuxième grandeur physique (Iⱼ ; dVⱼ/dt ; dIⱼ/dt) et d'une valeur ou d'une forme d'onde respective parmi la pluralité de valeurs ou de formes d'onde de la troisième grandeur physique (Vᵢ ; Iᵢ ; Vᵢ)

3. Méthode selon la revendication 1 ou 2, la détermination (200) d'une deuxième matrice (RM ; CM ; LM) de valeurs de la première grandeur physique (R ; C ; L) comprenant :
- l'alimentation de valeurs ou de formes d'onde supplémentaires de la deuxième grandeur physique (I'ⱼ; dV'ⱼ/dt; dI'ⱼ/dt) une par une aux éléments capteurs (3 ; 13) respectifs ; et
- pour chaque valeur ou forme d'onde supplémentaire de la deuxième grandeur physique (I'ⱼ; dV'ⱼ/dt; dI'ⱼ/dt), la mesure d'une pluralité respective de valeurs ou de formes d'onde supplémentaires de la troisième grandeur physique (V'ᵢ; I'ᵢ; V'ᵢ) par l'intermédiaire de tous les éléments capteurs (3 ; 13) et le calcul d'une pluralité respective de valeurs de la deuxième matrice (RM ; CM ; LM), chacune selon la valeur ou la forme d'onde supplémentaire de la deuxième grandeur physique (I'ⱼ; dV'ⱼ/dt; dI'ⱼ/dt) et d'une valeur ou d'une forme d'onde respective de la pluralité de valeurs ou de formes d'onde supplémentaires de la troisième grandeur physique (V'ᵢ; I'ᵢ; V'ᵢ).

4. Méthode selon l'une quelconque des revendications 1 à 3, la sélection (400) d'une valeur propre minimale (λm) à partir desdites valeurs propres (λ) comprenant :
- le classement desdites valeurs propres (λ) par ordre décroissant et l'expression de cette tendance décroissante dans une échelle logarithmique ;
- la sélection de la valeur propre la plus faible précédant un changement de pente de la tendance décroissante sur l'échelle logarithmique.

5. Méthode selon l'une quelconque des revendications 1 à 4, ladite première grandeur physique étant la résistance électrique (R), la grandeur physique d'intérêt étant la conductivité électrique (σ), la deuxième grandeur physique étant le courant électrique (I), la troisième grandeur physique étant la tension électrique (V) et les éléments de détection étant des électrodes (3) en contact avec les points de surface (4) respectifs.

6. Méthode selon l'une quelconque des revendications 1 à 4, ladite première grandeur physique étant la capacité électrique (C), la grandeur physique d'intérêt étant la permittivité diélectrique (ε), la deuxième grandeur physique étant la dérivée temporelle de la tension électrique (dV/dt), la troisième grandeur physique étant le courant électrique (I) et les éléments capteurs étant des électrodes (3) en contact avec des points de surface (4) respectifs.

7. Méthode selon l'une quelconque des revendications 1 à 4, ladite première grandeur physique étant la capacité électrique (C), ladite grandeur physique d'intérêt étant la perméabilité magnétique (µ), ladite deuxième grandeur physique étant la dérivée temporelle du courant électrique (dI/dt), ladite troisième grandeur physique étant la tension électrique (V) et lesdits éléments capteurs étant des pôles magnétiques (13) faisant face à des points de surface (4) respectifs.
